(19) **Europäisches Patentamt**
**European Patent Office**
**Office européen des brevets**

(11) **EP 3 283 647 B1**

(12) **EUROPEAN PATENT SPECIFICATION**

(45) Date of publication and mention
of the grant of the patent:
**07.11.2018 Bulletin 2018/45**

(51) Int Cl.:
*C12Q 1/68* (2018.01)   *G06F 19/18* (2011.01)
*G06F 19/22* (2011.01)

(21) Application number: **16734594.1**

(22) Date of filing: **23.06.2016**

(86) International application number:
**PCT/EP2016/064604**

(87) International publication number:
**WO 2017/220156 (28.12.2017 Gazette 2017/52)**

(54) **A METHOD FOR NON-INVASIVE PRENATAL DETECTION OF FETAL CHROMOSOME ANEUPLOIDY FROM MATERNAL BLOOD**

VERFAHREN ZUM NICHT-INVASIVEN PRÄNATALEN NACHWEIS VON FÖTALER CHROMOSOMENANEUPLOIDIE AUS MÜTTERLICHEM BLUT

METHODE DE DETECTION PRENATALE NON-INVASIVE D'ANEUPLOIDIE CHROMOSOMIQUE FETALE DANS LE SANG MATERNEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.02.2018 Bulletin 2018/08**

(73) Proprietor: **Trisomytest, s.r.o.**
**841 04 Bratislava - Karlova Ves (SK)**

(72) Inventors:
• **DURIS , Frantisek**
**010 01 Zilina (SK)**
• **BUDIS, Jaroslav**
**851 01 Bratislava (SK)**
• **SZEMES, Tomás**
**841 03 Bratislava (SK)**
• **MINÁRIK, Gabriel**
**951 48 Jarok (SK)**

(74) Representative: **Hak, Roman**
**Hak, Janecek & Svestka**
**Patent and Trademark Attorneys**
**U Pruhonu 5**
**170 00 Praha 7 (CZ)**

(56) References cited:
• **CHIU ROSSA W K ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES, NATIONAL ACADEMY OF SCIENCES, US, vol. 105, no. 51, 23 December 2008 (2008-12-23), pages 20458-20463, XP002620454, ISSN: 0027-8424, DOI: 10.1073/PNAS.0810641105 [retrieved on 2008-12-10] cited in the application & R. W. K. Chiu ET AL: "Supplementary Information for XP002620454 (Title: Noninvasive prenatal diagnosis of fetal chromosomal aneuploidy by massively parallel genomic sequencing of DNA in maternal plasma)", Proceedings of the National Academy of Sciences, vol. 105, no. 51, 10 December 2008 (2008-12-10), pages 20458-20463, XP055024153, US ISSN: 0027-8424, DOI: 10.1073/pnas.0810641105**

**(Cont. next page)**

- LAU TZE KIN ET AL: "Noninvasive prenatal diagnosis of common fetal chromosomal aneuploidies by maternal plasma DNA sequencing", THE JOURNAL OF MATERNAL FETAL & NEONATAL MEDICINE : THE OFFICIAL JOURNAL OF THE EUROPEAN ASSOCIATION OF PERINATAL MEDICINE, THE FEDERATION OF ASIA AND OCEANIA PERINATAL SOCIETIES, THE INTERNATIONAL SOCIETY OF PERINATAL OBSTETRICIANS, INFORMA HEALTHCA, vol. 25, no. 8, 1 August 2012 (2012-08-01) , pages 1370-1374, XP008164835, ISSN: 1057-0802, DOI: 10.3109/14767058.2011.635730 cited in the application
- A. J. SEHNERT ET AL: "Optimal Detection of Fetal Chromosomal Abnormalities by Massively Parallel DNA Sequencing of Cell-Free Fetal DNA from Maternal Blood", CLINICAL CHEMISTRY, vol. 57, no. 7, 1 July 2011 (2011-07-01), pages 1042-1049, XP055035087, ISSN: 0009-9147, DOI: 10.1373/clinchem.2011.165910 cited in the application
- H. CHRISTINA FAN ET AL: "Sensitivity of Noninvasive Prenatal Detection of Fetal Aneuploidy from Maternal Plasma Using Shotgun Sequencing Is Limited Only by Counting Statistics", PLOS ONE, vol. 5, no. 5, 1 January 2010 (2010-01-01) , page e10439, XP055026436, ISSN: 1932-6203, DOI: 10.1371/journal.pone.0010439

**Description**

Field of the invention

The invention relates generally to the field of non-invasive prenatal screening and diagnostics. The invention provides a method for detection of the presence or absence of aneuploidies, particularly trisomy of chromosome 21, 18 and 13, in fetus from the blood sample taken from mother in early stage of pregnancy.

Background of the invention

Nowadays, prenatal testing is an integral component of obstetric practice. The primary aim of prenatal testing is screening for fetal aneuploidies, such as trisomy of chromosome 21 (T21, Down syndrome), trisomy 18 (T18, Edwards syndrome), and trisomy 13 (T13, Patau syndrome). Although the majority of fetuses with aneuploidy result in termination during the development of the fetus, T21 has the highest survival rate, therefore, the prenatal detection of T21 is considered the most important prenatal genetic test for the purpose of prenatal screening or diagnostics. Reliable invasive prenatal tests are available, however, because of their risky nature, they are currently preformed only in high-risk pregnancies. Developing a reliable method for non-invasive prenatal testing (NIPT) for fetal aneuploidies, particularly trisomies, seems to be recent challenge of the utmost importance in prenatal care.

From medical point of view, also other trisomies, for example trisomy of chromosome 8, 9, 16 or 22 could be interesting. Also prenatal testing for fetal monosomies, for example monosomy of X chromosome (Turner's syndrome), could be important.

The discovery of circulating cell-free fetal DNA (cffDNA) in maternal blood (*Lo et al.*, 1997) has offered the possibility for developing non-invasive processes that use fetal nucleic acids from a maternal peripheral blood sample to determine fetal chromosomal abnormalities. CffDNA constitutes approximately less than 10% of the total circulating cell-free DNA (cfDNA) in maternal plasma, however, it has recently been found that the entire fetal genome, in the form of cffDNA, is present in maternal blood and thus it is very promising material for NIPT.

Several documents *(Chiu et al., 2008, Fan et al., 2008, Chiu et al, 2011, Sehnert et al., 2011, Lau et al., 2102 and Bianchi et al., 2012,* EP 2183693*,* EP 2366031*,* US 8296076) disclosed the methods in which sequencing of DNA in maternal blood is used to obtain information on aberrant chromosome dosage in fetus. All methods mentioned above use analysis of the total cfDNA in maternal plasma without the need to isolate fetal-specific DNA, cffDNA. These methods are based on the detection of the extra copy of chromosome to distinguish normal cases from trisomy cases. In case of a fetus with trisomy, the number of copies of trisomic chromosome in the maternal blood is slightly higher in comparison with other chromosomes. Analogous approach can be used for detection of monosomies, wherein the number of copies of monosomic chromosome is lower. The advent of mass parallel DNA sequencing (MPS) permitted sequencing of extremely large quantities of DNA molecules and thus next-generation DNA sequencing (NGS) have recently been used to detect non-invasively fetal aneuploidy from maternal blood.

Generally, the detection of fetal trisomy using NGS is done through the following process. First, a short region at one end of each DNA molecule of maternal plasma is sequenced and the obtained sequence reads are mapped against the reference human genome to determine the chromosomal origin of each sequence. Next, the amount of the tags (i.e. mapped reads) from the chromosome of interest (e.g. chromosome 21) is compared with some kind of reference chromosome (i.e. with the amount of tags of another individual chromosome or selected group of chromosomes) usually by means of a ratio of such values and the resulting ratio is compared between the examined sample and euploid control samples.

EP 2183693 (Lo, Y.M.,D. et al.*, assigned to Chinese University of Hong Kong, HK*) disclosed a method in which respective amounts of a clinically relevant chromosome and of background chromosome are determined from results of MPS and calculated percentage representation of sequences mapped to the chromosome 21. It was found that such a percentage representation is significantly higher in a sample of pregnant woman carrying a trisomy 21 fetus comparing to a woman with normal fetus. No z-score was determined as a decisive value in this method.

US 8296076 (Fan, Ch. H.M. and Quake, S.R.*, assigned to The Board of Trustees of the Leland Stanford Junior University, US*) disclosed a method of fetal aneuploidy diagnostics based on sequencing. By counting the number of tags mapped to a predefined window in each chromosome, the over- or under-representation of any chromosome in maternal plasma DNA contributed by an aneuploid fetus can be detected.

The methods of *Chiu et al., 2008, Chiu et al., 2011, Sehnert et al., 2011, Lau et al., 2012* are based on whole genome sequencing using MPS and z-score determination. Although z-score is widely accepted as the standard parameter used for detection of aneuploid samples, there are differences in its determination. *Chiu et al., 2011* disclosed an approach using tags mapped to all chromosomes used as reference for z-score determination, *Lau et al., 2012* disclosed an approach using tags mapped to some specific chromosome, e.g. 14 as reference for T21, and *Sehnert et al., 2011* chose chromosome 9 to be the optimal internal reference for the chromosome 21.

Generally, there are three main stages of the determination of aneuploidy of fetus from a maternal blood, plasma or serum sample: 1) preparation of DNA sample and DNA library, 2) sequencing, and 3) analysis of the sequence data. Sequencing made remarkable progress in the past few years, however, in the first and the third stage there is a space for improving that might have low cost and big impact on the quality of the testing.

There are various approaches how to improve the first stage, as disclosed for example in EP 2366031 (Rava, R.P. et al*., assigned to Verinata Health, Inc.,* US). The document disclosed a method for prenatal screening and diagnostics of fetal chromosomal aneuploidy on the basis of NGS comprising a novel protocol for preparing sequencing libraries from a maternal sample. The novel approach in preparing sequencing libraries comprises the consecutive steps of end-repairing, dA-tailing and adaptor ligating said nucleic acids, and wherein said consecutive steps exclude purifying the end-repaired products prior to the dA-tailing step and exclude purifying the dA-tailing products prior to the adaptor-ligating step. The method allows for determining copy number variations (CNV) of any sequence of interest. No z-score was determined as a decisive value in this method.

Another improvement was disclosed in WO 2011/051283 (Benz, M. et al*., assigned to Lifecodexx, AG, DE*). The method for non-invasive diagnosis of chromosomal aneuploidy disclosed therein is improved by the enrichment and quantification of selected cfDNA sequences in a maternal blood sample.

In spite of existence of several methods for non-invasive detection of fetal aneuploidy there is still a need for improved method or alternative method that would be at least as sensitive and specific as the present methods.

Description of the invention

The present invention provides alternative and reliable method that is applicable to the practice of non-invasive prenatal screening for aneuploidies, such as trisomies or monosomies, preferably for trisomy of chromosome 13, 18 or 21. The present method is improved over the prior art methods especially in the part of the processing of the sequencing data. Due to the novel and inventive approach this method provides an alternative z-score method, that affords better distinction between euploid and aneuploid samples, i.e. improves the reliability of the test. Additionally, the method needs relatively low amount of sequencing data and therefore such a method is relatively cheap and would be affordable even for the small healthcare institutions.

The following description will explain the main features of the method of the present invention; however, it does not imply that the invention must include all features and aspect described herein. The scope of the protection will be defined by the patent claims attached to this description. The skilled person will get full understanding of the present invention from the following description together with the examples, where some specific features and aspects will be explained in more details, and accompanying figures.

The technical and scientific terms used herein have the same meaning as commonly understood by the persons skilled in the art of medicine, molecular biology, genetics, bioinformatics and prenatal diagnostics, unless defined here otherwise.

*Summary of the method according to the present invention*

The present invention relates to the method for determining aneuploidy of fetus from a maternal blood sample comprising a mixture of fetal and maternal nucleic acid molecules, wherein the mixture of fetal and maternal nucleic acid molecules are cell-free DNA molecules, as defined in claim 1. Said method comprises four main stages: 1) treating samples of maternal blood, 2) preparation of DNA sample and DNA library, 3) sequencing, and 4) processing the sequence data to obtain a value that have a predictive significance. The essential part of the method is preparation and processing of the training data, i.e. processing of the set of euploid samples that are processed in the same way as test samples. The output from the training samples processing serve as a reference data in step 4) mentioned above. The term blood sample is used in broad meaning and comprises blood as such as well as plasma or serum sample.

The method according to the present invention can be applied to any aneuploidy, such as monosomy or trisomy, for example aneuploidy of chromosomes 8, 9, 13, 16, 18, 21, 22 and X, particularly trisomy of chromosomes 13, 18 and 21.

The main improvement and advantage of the present method lies in the step of processing and analysing the sequence data. The determination of the value (z-score) that is decisive for the method (comparing of this particular value with a preset cut-off value allows to determine the presence of aneuploidy, as trisomy or monosomy) is performed on the basis of a model of multinomial distribution of tags to chromosomes or bins of human genome. As it is demonstrated in the examples, z-score values of trisomic samples determined by the method of the present invention are significantly higher than values determined by any of the prior art method (for T21 samples we obtained median difference of z-score values -1.895, -1.595, -2.485 when comparing *Chiu et al., 2008, Sehnert et al., 2011, Lau et al.,* 2012, respectively, with our method with difference p-value $< 0.05$ for all prior art according to Dunn's multiple comparisons test), while values of normal (non-trisomic) samples are substantially identical (with respect to chromosome 21 we obtained median difference from our method -0.15, -0.05, -0.21 for *Chiu et al., 2008, Sehnert et al., 2011, Lau et al., 2012,* respectively), which means that the distance of z-scores of trisomic samples from the cut-off value is for the present invention increased when compared with the prior art methods while the distance of the euploid samples from this cut-off value is basically preserved. Since this distance determines how sure we are with our diagnosis (for trisomic samples, the larger the better), the present invention improves the quality of the test.

The starting point of the present method is processing of the maternal sample, which is peripheral blood, thus the method is non-invasive. The peripheral blood comprises cell free DNA (cfDNA) that is a mixture of maternal and fetal DNA (cffDNA). CffDNA is what matters; therefore fetal DNA can be enriched (by selection of the shorter fragment, either *in silico* or physical selection). Then, DNA sample (still comprising both maternal and fetal DNA, i.e. it is a mixed sample) is subjected to the massively parallel sequencing, as performed by NGS approach, to obtain huge number of short sequence reads. These reads serve as tags, i.e. they are mapped to a certain genomic region or chromosome. For the purpose of this method, the whole human genome (24 chromosomes together representing DNA with over 3 x $10^9$ bp) is divided into non-overlapping 1 Mbp bins (i.e. approx. 3 x $10^3$ bins), and the present method considers both tags mapped to chromosomes as well as these bins. Then, simply speaking, by counting the number of tags mapped to the particular bins of interest (e.g. bins associated with potentially triploid chromosome like chromosome 21), and by comparing this number with number of tags mapped to some kind of reference chromosome (or a set of reference chromosomes or their parts, i.e., a particular set of reference bins), the irregular representation of the chromosome of interest or its parts can be detected.

The essential part of the method is preparation and processing of the training samples, i.e. processing of the set of euploid samples that are processed in the same way as test samples. The set of training data serve as a base for selection of the reference chromosomes or their parts. The selection of proper reference as well as sufficiently random training set is of the utmost importance as stemmed from our own experiments as well as from prior art documents cited above. Therefore, our approach comprises novel and improved method of selection of internal reference, specifically internal reference chromosomes and internal reference bins.

Further, the present method comprises determination of the z-score value based on a novel approach that has not been used till this time. Briefly, the z-score is calculated from the mean and standard deviation values determined on the basis of multinomial distribution of tags to the bins. Finally, the comparison of the z-score with the preset limit value (e.g. 3) is indicative whether or not the aneuploidy exists.

The present multinomial approach is, in principle, alternative z-score method; however, it is based on the data significantly more general than prior art methods. More particularly, the first part of the improvement of the present method lies in more general selection of reference set of chromosomes. Not only we consider for the internal reference any combination of chromosomes (excluding combinations with chromosomes 13, 18 and 21 because of the potential trisomy), we also allow there to be only parts for the chromosomes, i.e., bins (with 1 Mbp resolution). In this way we can better characterize the euploid population and thus more easily find deviations, i.e. aneuploid samples. Thus, the first benefit of our approach lies in that the internal reference can be any set of chromosomes or parts of the chromosomes with the resolution of 1 Mbp bins (instead of one specific chromosome or all autosomes as in prior art methods). The other benefit of the present method is that our multinomial approach is based on mathematically sound and complex modelling of the distribution of tags to the bins and of the consecutive ratio of the aneuploid, e.g. trisomic, chromosome to the internal reference bins. Multinomial approach leads to significantly higher z-score for trisomic samples when compared to prior art methods, while maintaining no substantial change in the z-scores of euploid samples. Thus, the reliability of the test is improved. Additionally, the present method allows the estimation of fetal fraction together with the error of this estimation. While the estimation of fetal fraction was presented before in *Rava et al., 2014, Hudecova et al., 2014,* and *Yu et al., 2014,* no error of this estimation was presented as of yet. Furthermore, the present method allows the estimation of the minimal number of mapped tags with which an aneuploid, e.g. trisomic sample with certain fetal fraction is expected to exhibit a z-score at least some predetermined value (e.g. cut-off value). This information can be valuable for deciding cases with low fetal fraction and close-to-cut-off value of z-score.

There is a practical requirement to automatize the methods of the prenatal tests or diagnostics. The method of the present invention can be largely automatized. At least the bioinformatics part of the method (i.e. processing of sequencing data and all subsequent determinations and calculations) may be performed using any suitable computing system, such as for example PC equipped with a processor, peripheral input/output devices (e.g. ports, interfaces), memories (e.g. system memory, hard disk), keyboard, monitor, mouse etc. Preferably computing system may be in data communication with the sequencing system providing the sequence data, preferably in the form of plurality of sequence reads (by a wire or wireless networking, bluetooth, internet, cloud etc.). It means that the computing system is configured for receiving sequence data from the sequencing system. The suitable computing systems as well as means for connection with sequencing system are well known to the persons skilled in the art.

At least part of the method, specifically the bioinformatics part of the method, can be implemented as a software code, i.e. a plurality of instructions (computer programme) to be executed by a processor of a computing system. The code may be comprised in the computer readable medium for storage or transmission such as for example RAM, ROM, hard-drive, SDS, CD, DVD, flash memory etc. Furthermore, the code may be transmitted via any suitable wired, optical or wireless network, for example via internet. For example the whole computer programme can be downloaded by the user (customer) via the internet. Therefore the present invention relates also to a computer program product comprising a computer readable medium comprising a plurality of instructions for controlling a computing system to perform all steps of the method of the invention following the step (b) of performing a random sequencing.

The computer program mentioned above can be preferably introduced into computer of the sequencing system.

The subject matter of the present invention is defined in the claims 1- 12 as attached.

The features and advantages of the present invention will be understood by the person skilled in art from the following detailed description, examples and figures.

*Detailed description of the method*

<u>*THE METHOD*</u>

The method is a z-score method based on the model of multinomial distribution of mapped tags to the bins. Note that in this section we write, as example, mostly about trisomy, especially of the chromosome 21, however, the method can be applied to any aneuploidy, monosomy or other trisomy, particularly trisomy of chromosome 13 and 18, in an analogous manner.

Prerequisite of the test sample processing is the selection and processing of the proper training set (i.e. set of training samples) in order to choose proper internal reference for the evaluation of the test samples. Generally, the training set should include euploid female samples as well as euploid male samples and the samples must be treated necessarily in the same way as the test samples.

*TRAINING SAMPLES*

Euploid male and female samples are selected to form the training set denoted as $T_h$. For each sample from the training set the following steps are performed:

    1. Collection and treatment of blood samples.

    2. DNA isolation.

    3. Sequencing.

    4. For each sample from the training set apply the following steps of sequence data processing:

        (a) Raw sequence reads are mapped to the unmasked reference human genome (hg19, Genome Reference Consortium Human Build 37 (GRCh37), Feb. 2009, GenBank assembly accession: GCA_000001405.1, RefSeq assembly accession: GCF_000001405.13) using a Bowtie2 algorithm (*Langmead and Salzberg, 2012*).

        (b) Only sequence reads that could be mapped to just one genomic location with at most one mismatch are retained.

        (c) GC correction is performed according to *Liao et al., 2014* (in our case without the intrarun normalization).

        (d) Optional: Filter reads according to their size (e.g. < 150bp) (*Minarik et al., 2015*).

        (e) The reference genome hg19 is split into 1 Mbp subsequent and non-overlapping bins with the mapped DNA tags distributed to bins according to their mapping location.

The size filtering in step 4d can be used to artificially increase fetal fraction and by this z-score of trisomic samples as well. However, this is usually at the cost of lowering the sample overall tag count as tags longer than the considered threshold are discarded (for details see *Minarik et al., 2015*).

Further, the internal reference bins, denoted as IRB, must be chosen before the determination of the z-score value of the test sample, and this is done by means of a choice of internal reference autosomes, denoted as IRA, using training set $T_h$ and a genetic algorithm. Therefore the method comprises the steps:

    5. Choosing the internal reference autosomes, IRA, using the training set $T_h$.

(a) Any combination of autosomes (excluding combinations with autosomes 13, 18, and 21 because of the potential aneuploidy) is considered as a potential internal reference (together 524 287 candidate combinations).

(b) For each potential internal reference combination the coefficient of variation (CV) is calculated (see further *Calculation of coefficient of variation of candidate internal reference autosomes combination* below).

(c) The candidate internal reference combinations of autosomes are ordered according to their CV value in increasing order.

(d) Internal reference combinations whose CV value is less than or equal to 1.1 multiple of the overall lowest CV value are chosen as IRA. Preferably at least one hundred (i.e. the first hundred) of these combinations are chosen (these are the best internal reference autosome combinations in terms of CV values).

6. Choosing the internal reference bins, IRB, by a genetic algorithm using the training set *Th* (see further *Choosing internal reference bins* below).

*Calculation of coefficient of variation of candidate internal reference autosomes combination*

Let $M = (m_{i,j})_{kx22}$ be train matrix associated with the train set $T_h$, where $k$ is the size of $T_h$ and $m_{i,j}$ holds the number of tags mapped (and GC corrected) to the $j^{th}$ autosome for $i^{th}$ sample of $T_h$, and let $ir = \{0, 1\}^{22}$ be a candidate internal reference combination ($ir[i] = 1$ when $i^{th}$ autosome, $i \in \{1, 2, ..., 22\}$, was chosen for internal reference, otherwise $ir[i] = 0$). Also, let $M_i$ denote $i^{th}$ row of the matrix $M$. The normalized value of the $i^{th}$ sample (i.e., $i^{th}$ row of the matrix $M$) is defined as $m_{i,x}/M_i \cdot ir$, where x is the aneuploid chromosome we want to test. We denote the normalized value of sample $i$ as $v_i$. Let $V_{ir} = (v_1, v_2, ... , v_k)$. Then, the *CV* value of the candidate internal reference combination *ir* is defined as

$$CV_{ir} = 100 \cdot \frac{stdev(V_{ir})}{mean(V_{ir})} \qquad \text{(Eq. 1)}$$

*Choosing the internal reference bins* (genetic algorithm)

Let $M = (m_{i,j})_{kxt}$ be a matrix associated with the train set $T_h$, where $k$ is the size of $T_h$, $t$ is the number of 1Mbp bins that cover hg19 genome, and $m_{i,j}$ holds the number of tags mapped to the $j_{th}$ bin for $i_{th}$ sample of $T_h$. Also, let $M_i$ denote $i^{th}$ row of the matrix $M$. A candidate combination of internal reference bins (IRB) is any combination of the columns of the matrix $M$ excluding the combinations that include bins associated with chromosomes 13, 18 and 21 (these bins should not be in the selected IRB because of the potential trisomy; other trisomies are very rare). Let let $ir = \{0, 1\}^t$ denote such candidate IRB ($ir[j] = 1$ when $j^{th}$ bin, $j \in \{1, 2, ..., t\}$, was chosen for internal reference, otherwise $ir[j] = 0$). Furthermore, let binary vector $v_{21}$ denote bins associated with chromosome 21 analogously to vector *ir* (similarly for chromosomes 13 and 18 or other aneuploidies). For each candidate IRB we calculate fractional bin value $FB_i$ for each sample in $T_h$ from the $i^{th}$ row of the matrix $M$ as

$$FB_i = \frac{M_i \cdot v_{21}}{M_i \cdot ir}, \qquad \text{(Eq. 2)}$$

where · represents the dot product. Let $V_{ir} = (FB_1, FB_2, ..., FB_k)$ hold fractional bin values for all samples in $T_h$. We assign each candidate IRB Student range coefficient (*SRC*) that is given by

$$SRC_{ir} = \frac{\max(V_{ir}) - \min(V_{ir})}{stdev(V_{ir})}. \qquad \text{(Eq. 3)}$$

The *SRC* value describes the spread of the *FB* values of the training set. In addition to *SRC* value, we also assign each candidate IRB a CV value that is calculated analogously to the process of *Calculation of coefficient of variation of candidate internal reference autosomes combination.* We want to find such IRB that has *SRC* and *CV* values as small as possible. However, the set of all IRB candidates has approximately $2^{3000}$ members and so it is not possible to calculate the *SRC* and CV values for all of them. Therefore, to select a good IRB we use a genetic algorithm (genetic algorithms are well known to the person skilled in the art, and we used a program DEAP as published by *Fortin et al.,*

*2012.* More particularly, the initial population of IRB candidates used in the genetic algorithm consists of two sets. The first set is a random selection of bins from all autosomes, excluding the bins from autosomes 13, 18 and 21 because of the potential trisomy, while the second set is based on the previously determined best internal reference autosomes (IRA). In this way, there are already good individuals in the population of candidate IRBs (that is, those from the IRA set), and the genetic algorithm can only enhance the previous combinations from IRA selection, for example, by selecting only some bins of the IRA combinations.

The genetic algorithm will possibly not select the best IRB in terms of *SRC* and CV values, but the resulting IRB will be (often considerably) better than IRA. This is so because IRB allows there to be only parts of the chromosomes in the reference as opposed to the whole chromosomes in the IRA case.

We would like to emphasize that the selected IRB depends on the chosen chromosome of interest (e.g. chromosome 21), samples in the training set as well as some chance due to the nature of the genetic algorithms. Thus, multiple runs of this part of process will most likely result in different IRB. For this reason it is advisable to repeat this process multiple times (the number of times is limited only by the time we are willing to spend on this part of the process) and to chose the best IRB so far found.

*TEST SAMPLE*

The test sample is processed exactly in the same way as the training samples in steps 1 to 4 as described in details above. Further, using the internal reference bins, IRB, as determined above, the following steps are performed:

5. The mapped tags of the test sample are transformed into fractional bin value (*FB*) using the chosen IRB set (see *Choosing internal reference bins*)

$$\mathrm{FB} = s_{21}/s_{\mathrm{IRB}} \qquad (\text{Eq. 4})$$

wherein $s_{21}$ denotes the number of tags of the test sample mapped to the bins of chromosome 21 (or other chromosome of interest) and $s_{\mathrm{IRB}}$ denotes the number of tags of the test sample mapped to IRB bins.

6. Multinomial mean $\mu(n)$ and standard deviation $\sigma(n)$ for the test sample are calculated (see further *Calculation of multinomial mean and standard deviation*)

7. The test sample is evaluated (see further *Evaluation of the test sample*)

*Calculation of multinomial mean and standard deviation*

First note that the by multinomial mean and standard deviation we do not mean the mean and standard deviation of the multinomial distribution. Rather, it is the mean and standard deviation of a new random variable derived from the multinomial distribution that we define in the section *The mathematical model* further in this description.

Let IRB be a set of chosen internal reference bins. Let $T_h$ be a set of train euploid samples of size $k$. Let $M = (m_{i,j})_{\mathrm{kxt}}$ be a matrix associated with the train set $T_h$, where $k$ is the size of $T_h$, $t$ is the number of 1 Mbp bins that cover human genome hg19, and $m_{i,j}$ holds the number of tags mapped to the $j^{th}$ bin for the $i^{th}$ sample of $T_h$. Let $m_i$, $i \in \{1, 2, ..., k\}$, denote the sum of the $i^{th}$ row of the matrix $M$. Let $P = (p_{i,j})_{\mathrm{kxt}}$ be matrix such that $p_{i,j} = m_{i,j}/m_i$, $i \in \{1, 2, ..., k\}$, $j \in \{1, 2, ..., t\}$, where $m_{i,j}$ is an element from the matrix $M$. Furthermore, let $p_j$, $j \in \{1, 2, ..., m\}$, denote the mean value of the $j^{th}$ column of the matrix $P$, and let $p = (p_1, ..., p_t)$. We interpret the value $p_j$, $j \in \{1, 2, ..., m\}$, as a probability that a DNA fragment will map onto the $j^{th}$ bin. Observe that this vector is given by the training set $T_h$, and it is also the only values our method requires to train. We observed that these values change with different laboratory processing of the samples, so the training samples of $T_h$ and the test samples need to be prepared in the same way. Let $P_x$, $X \in \{13, 18, 21\}$ depending on the trisomy we would like to test, and $P_{IRB}$ be the sum of probabilities for bins associated with $X^{th}$ chromosome and IRB, respectively. We define the multinomial mean $\mu(n)$ and multinomial standard deviation $\sigma(n)$ as

$$\mu(n) = \frac{P_X}{P_{IRB}}\left(1 - (1 + P_{IRB})^n\right) \quad X \in \{13, 18, 21\}, \qquad (\text{Eq. 5})$$

$$\sigma(n) = \sqrt{\frac{W_1}{n} + \frac{W_2}{n^2}}, \qquad (\text{Eq. 6})$$

where

$$W_1 = \left(\frac{P_X}{P_{IRB}}\right)^2 \left(\frac{1}{P_X} + \frac{1}{P_{IRB}}\right), \qquad (\text{Eq. 7})$$

$$W_2 = \frac{P_X}{1 - P_{IRB}} \left(\frac{1}{P_{IRB}}\right)^3 \frac{1 - P_X - P_{IRB}}{1 - P_{IRB}}, \qquad (\text{Eq. 8})$$

and n is the total number of the mapped tags (now including the tags from bins associated with chromosomes 13, 18 and 21) of the sample that we want to test. Note that test sample with different n have different multinomial mean and standard deviation. In this way the test is personalised for each sample.

*Evaluation of the test data*

Let *FB* be the fractional bin value of a sample we want to test (see *Choosing the internal reference bins*). Let *n* be the total number of the mapped DNA tags (including tags from bins associated with chromosomes 13, 18 and 21) for this sample. Given a trisomy case we would like to examine, we calculate the appropriate multinomial mean $\mu(n)$ and multinomial standard deviation $\sigma(n)$ (see above *Calculation of multinomial mean and standard deviation*). Subsequently, for our test sample we calculate its z-score

$$z = \frac{FB - \mu(n)}{\sigma(n)}, \qquad (\text{Eq. 9})$$

and if z is higher then preset value, the sample is marked by present method as trisomic (usually for z > 3).

*Fetal fraction*

Additionally, the approximate fetal fraction *f* of the test sample is given by

$$f \approx 2\frac{FB \cdot P_{IRB} - P_{21}}{P_{21}}, \qquad (\text{Eq. 10})$$

where $P_{21}$ and $P_{IRB}$ are from *Calculation of multinomial mean and standard deviation* (analogously for other trisomies) and *FB* is fractional bin value from *Choosing the internal reference bins.*

*The error of the fetal fraction estimation*

Let *p* = ($p_1$, ..., $p_t$) be from *Calculation of multinomial mean and standard deviation*. Let Q(f) = (q_1(f), q_2(f), q_3(f), ..., q_t(f)) where

$$q_i(f) = \begin{cases} \frac{p_i + \frac{f}{2}p_i}{1 + \frac{f}{2}S_{21}} & \text{for bins assoc. with } 21^{st} \text{ chrom.} \\ \frac{p_i}{1 + \frac{f}{2}S_{21}} & \text{for other bins,} \end{cases} \qquad (\text{Eq. 11})$$

where S_21 is the sum of p_j associated with the chromosome 21. The i ranges from 1 to t, and f is a variable that can range from 0 to 1. For other trisomies the equation is analogous. For monosomy the equation changes to

$$q_i(f) = \begin{cases} \frac{p_i - \frac{f}{2}p_i}{1 + \frac{f}{2}S_{21}} & \text{for bins assoc. with chromosome 21} \\ \frac{p_i}{1 + \frac{f}{2}S_{21}} & \text{for other bins,} \end{cases} \qquad (\text{Eq. 12})$$

We transform the vector Q(f) to vector Q'(f) = (Q_X, Q_IRB, 1 - Q_X - Q_IRB) where Q_X is the sum of the q_i(f) values associated with the chromosome 21 or other aneuploid chromosome, and Q_IRB is the sum of the q_i(f) values associated with the IRB bins. Note that until we define the value of f, the vectors Q(f) and Q'(f) are symbolic. Using the values Q_X and Q_IRB instead of P_X and P_IRB, we define the values $\mu(n, f)$ and $\sigma(n, f)$ analogously to values $\mu(n)$ and $\sigma(n)$ from *Calculation of multinomial mean and standard deviation.* Observe, that we write $\mu(n, f)$ instead of $\mu(n)$ because we still do not set the value of f.

Let *FB* be the fractional bin value of a sample we are currently working with (see *Choosing the internal reference bins*). By solving the equation

$$FB - \mu(n, f) = 2^* \, \sigma(n, f) \qquad \text{(Eq. 13)}$$

for f (recall that n is the test sample's overall tag count), we get the lower bound on the fetal fraction of the test sample. By solving the equation

$$FB - \mu(n, f) = - 2^* \, \sigma(n, f) \qquad \text{(Eq. 14)}$$

for f, we get the upper bound on the fetal fraction of the test sample.

The nature of these bounds is as follows. Since the sequencing is a random process in the sense that the sequenced fragments are chosen randomly, it can happen that two sequencing runs of the same sample will end up with different number of sequenced fragments from, say, chromosome 21. For this reason the z-score of a sample can vary with different sequencing runs (given the same choice of IRB), and the same holds for the derived fetal fraction. These bounds are set in such a way that 95% of sequencing runs of the same sample is expected to result in fetal fraction within these bounds. Thus, for the previously calculated fetal fraction f, these bounds give us the likely margin of error for this value.

*Minimal tag count for reliable prediction*

For euploid samples the expected fractional bin (*FB*) value is approximately $p'_1 / p'_2$ (by *Theorem 1*). For a T21 sample, as example, with fetal fraction *f* this *FB* value is approximately equal to $(1 + f/2) \, p'_1 / p'_2$ because in trisomic samples the parameter $p'_1$ is increased by the amount $f/2 \, p'_1$ coming from the third copy of fetal chromosome 21 while other chromosomes do not change (assuming the sample does not have other trisomies present). There is also a normalising factor $1/(1 + f/2 \, p'_1)$ because by increasing the probability parameter $p'1$ we violated the equation $p'_1 + p'_2 + p'_3 = 1$.

However, this factor cancel out in the fraction $p'_1 / p'_2$. Thus, by *Theorems 1* and 2 (see further the expected z-score of a trisomic sample with fetal fraction *f* is (note that we neglected the term $W2/n_2$ from *Theorem 2* for the sake of simplicity)

$$z = \frac{\frac{p'_1}{p'_2}\left(1 + \frac{f}{2}\right) - \frac{p'_1}{p'_2}}{\sqrt{\frac{1}{n}\left(\frac{p'_1}{p'_2}\right)^2 \left(\frac{1}{p'_1} + \frac{1}{p'_2}\right)}} = \frac{\frac{f}{2}}{\sqrt{\frac{1}{n}\left(\frac{1}{p'_1} + \frac{1}{p'_2}\right)}}. \qquad \text{(Eq. 15)}$$

We want z to be at least some k. Additionally, the z-scores of euploid samples are distributed around 0 on both sides (0 being the expected z-score), and the same holds for trisomic samples (of course, such z-scores would be distributed around different mean value). Hence, it is possible for a trisomic sample that its fractional bin value FB will not be equal to $(1 + f/2) \, p'_1 / p'_2$; it can be both lower or higher. Thus, we want to have

$$z = \frac{\frac{f}{2}}{\sqrt{\frac{1}{n}\left(\frac{1}{p'_1} + \frac{1}{p'_2}\right)}} = k + l, \qquad \text{(Eq. 16)}$$

where *k* is the z-score we would like to achieve and *l* corrects the possibility of the sample s scoring lower than expected (like in a case of euploid sample that has z-score less than 0). Since z-scores of euploid samples follow the standard normal distribution (this assumption was positively tested with statistical tests), 95% of z-scores of these samples should fall within the interval [-2; 2], so setting $l$ = 2 seems reasonable. By solving the last equation for n we obtain the required total count of mapped DNA fragments for the expected z-score at least k as

$$n = \frac{4(k+l)^2}{f^2}\left(\frac{1}{p'_1} + \frac{1}{p'_2}\right). \qquad \text{(Eq. 17)}$$

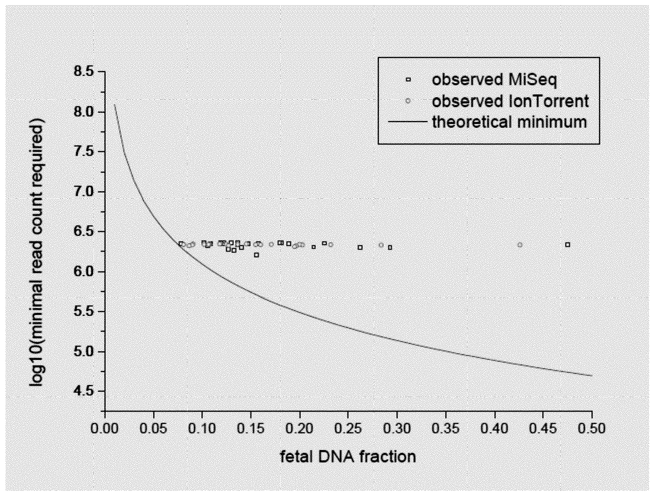

With $k$ = 4 and $l$ = 2, we get the minimal tag count around 1.24 million for fetal fraction $f$ = 0.1. More general plot is given in Figure 1.

### DEVELOPMENT OF THE MULTINOMIAL MODEL

### BIOLOGICAL MOTIVATION

It was observed that the fractional genomic representation (FGR) of a particular chromosome depends (among other things) on its size (*Chiu et al., 2008*; *Ehrich et al., 2011*). From this we can conclude, in a mathematical sense, that if a sample of maternal plasma is sequenced and these sequences are subsequently mapped and counted for each chromosome, then the outcome of this (random) process is a vector of 24 numbers, one for each chromosome, and we can view this vector as a random vector that is drawn from the multinomial distribution with parameters ($p_1$, $p_2$, ..., $p_{24}$; $n$), where $p_i$, $i \in$ {1, 2, ..., 24}, is probability that a particular sequenced DNA tag will map to the $i^{th}$ chromosome, and $n$ is the number of all mapped tags. Consequently, we formulate the following observation.

*Observation:* The sequencing data (i.e., mapped cfDNA tags) are multinomially distributed among the chromosomes with probability parameters strongly correlating with the chromosome lengths.

Going from chromosomes to 1 Mbp bins is a simple matter, only in this case the number of parameters $p_i$ is not 24 but the number of bins that cover human genome hg19 (we denoted this number as $t$).

### THE MATHEMATICAL MODEL

Let X be a random vector from the multinomial distribution. We define a new random variable $Y$ as a ratio of 2 or more elements of the multinomial random vector X. For example, for X=($x_1,x_2,x_3,...,x_{20}$) $Y$ can be defined as $Y = (x_1+x_2)/(x_2+x_3+x_{10})$. In order to model the situation described in *Biological motivation* we let the random vector X = ($x_1$, $x_2$, ..., $x_t$) stand for the number of tags mapped to 1 Mbp bins covering human genome hg19. For trisomy detection the numerator of $Y$ is the number of tags mapped to chromosome 21 (or 13 or 18), and the denominator of $Y$ is the sum of tags mapped to the *IRB* bins. However, this definition of $Y$ is needlessly general and complicated. This is because to get the value of $Y$ for a particular sample, it does not really matter, for example, to which particular IRB bin some DNA tag mapped; we only need to distinguish three classes of tags: 1) those mapped to a bin associated with the aneuploid chromosome, 2) those mapped to one of the internal reference bins, and 3) all others. Therefore, we can replace the multinomial random vector X with a new multinomial random vector X $'$= ($x'_1$, $x'_2$, $x'_3$), corresponding with the three mentioned classes, with the following new probability parameters given by

$$p'_1 = P_X, \quad X \in \{13, 18, 21\}, \qquad \text{(Eq. 18)}$$

$$p'_2 = P_{IRB}, \qquad \text{(Eq. 19)}$$

$$p'_3 = 1 - p'_1 - p'_2, \qquad \text{(Eq. 20)}$$

where $P_x$ and $P_{IRB}$ have been defined above in *Calculation of multinomial mean and standard deviation.* In this sense, we can now simplify the definition of $Y$ as $Y = x'_1 / x'_2$. Finally, in order to avoid problems with zero in the numerator of $Y$, we change the definition of $Y$ in the following way

$$Y = \frac{x'_1}{x'_2 + 1}. \qquad \text{(Eq. 21)}$$

Since the values assumed by random vector X $'= (x'_1, x'_2, x'_3)$ are of the order $10^4$ and larger, the +1 in the denominator of $Y$ has only a negligible effect. In the application of this model we need to know the values of $E[Y]$ and $Var[Y]$.

*Theorem 1.* Let X $'= (x'_1, x'_2, x'_3)$ be a random vector from the multinomial distribution with parameters $(p'_1, p'_2, p'_3; n)$. The expected value of the random variable $Y$ given by Eq. 21 is

$$E[Y] = \frac{p'_1}{p'_2}\left(1 - (1 - p'_2)^n\right). \qquad \text{(Eq. 22)}$$

*Theorem 2.* Let Let X $'= (x'_1, x'_2, x'_3)$ be a random vector from the multinomial distribution with parameters $(p'_1, p'_2, p'_3; n)$. For $n \geq 10^5$ and $1 > p'_1, p'_2 \geq 0.01$ the variance of the random variable $Y$ given by Eq. 21 can be approximated as

$$Var[Y] \approx \frac{W_1}{n} + \frac{W_2}{n^2} \qquad \text{(Eq. 23)}$$

where

$$W_1 = \left(\frac{p'_1}{p'_2}\right)^2\left(\frac{1}{p'_1} + \frac{1}{p'_2}\right), \qquad \text{(Eq. 24)}$$

$$W_2 = \frac{p'_1}{1 - p'_2} \cdot \left(\frac{1}{p'_2}\right)^3 \frac{1 - p'_1 - p'_2}{1 - p'_2}. \qquad \text{(Eq. 25)}$$

The error of this approximation is less than 0.01% of the true value.

A given test sample, characterized by its fractional bin value *FB,* is normalized using the values of $E[Y]$ and $(Var[Y])^{-1/2}$. In the currently used z-score methods (mentioned above and described below), if the sample's normalized value was grater than 3, it was considered trisomic. This cut-off value came from the assumption that normalized values are distributed according to the standard normal distribution. We also tested this assumption positively, that is, the random variable z given by Eq. 9 is standard normally distributed. For this reason, setting the cut-off value to 3, as is currently a standard, is appropriate for our method as well.

Brief description of the drawings

Figure 1: Estimation of the required sequencing depth of the circulating cell-free DNA in maternal plasma for the detection of fetal aneuploidy of 21st autosome as a function of fetal DNA fraction. The theoretical minimum is based on Eq. 17 and values k = 3 (minimal z-score for calling a sample trisomic), 1 = 2 (given by the dispersion of euploid z-scores around zero), and $p'_1$ = 0.0128, $p'_2$ = 0.128 (given by MiSeq train data). For IonTorrent the curve of the theoretical minimum is almost identical and is not plotted here. The plotted points display the observed T21 samples on MiSeq (squares) and IonTorrent (circles).

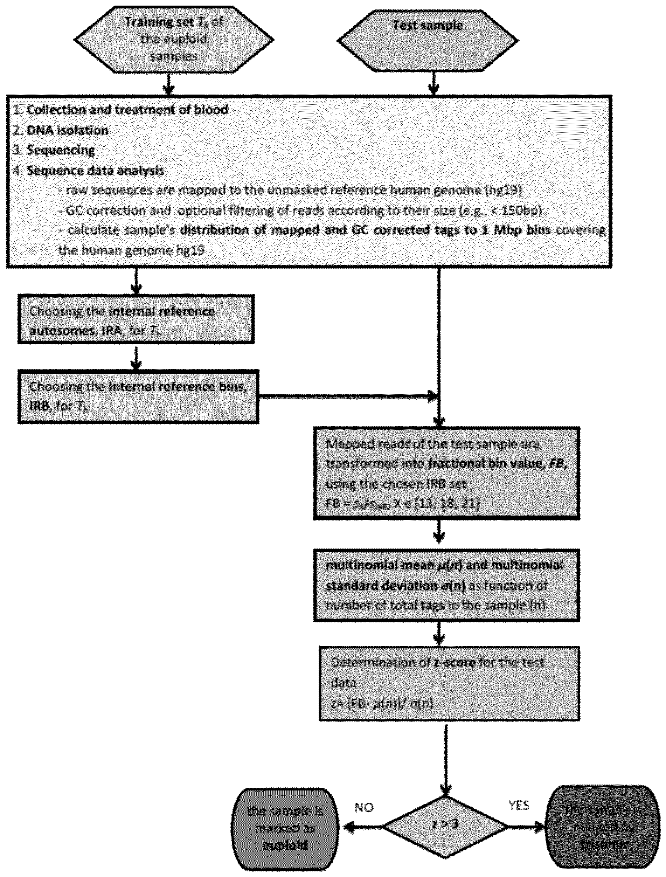

Figure 2: The flow chart scheme of the main steps of the method for non-invasive prenatal detection of fetal chromosome aneuploidy from the samples of maternal blood according to the present invention.

Examples

**Example 1**

***Sequencing techniques used in the realization of the invention***

*Sequence analysis*

Massively parallel sequencing is necessary for the application of the method according of the invention. The method was specifically developed and validated for small benchtop next generation sequencing systems to allow low initial costs for NIPT service lab setup. The method was validated on MiSeq system (Illumina, Inc., San Diego, CA, USA) and Ion Torrent PGM (Life Technologies Corp., San Francisco, CA, USA).

Commercially available sequencing devices together with corresponding protocols and reagents recommended by the supplier, were used in the illustrative example, however, the person skilled in the art is aware of number of various sequencing methods and their variations, which could be also used in practice of the present invention.

Illumina sequencing kit Version 3 and the kit MiSeq Reagent Kit v3 (MS-102-3003) were used.

In our experiments, on average $5.829\pm1.026$ million and $3.098\pm0.951$ million of raw reads were obtained after sequencing analysis on IonTorrent PGM and MiSeq, respectively. Because 200 bp single-end and 2x100 bp paired-end protocols were used on Ion Torrent PGM and Miseq, we were able to determine the native DNA fragment size distribution. The average read lengths of the reads $179\pm7$ bp and $172\pm7$ bp were recorded on IonTorrent PGM and MiSeq, respectively. To reach comparable conditions for comparison of the two tested benchtop NGS platforms in the validation step the gained raw read counts were normalized and the maximum 3 million of reads were randomly selected for all samples for consequent steps. After quality filtering of reads, read mapping, selection of uniquely mapped tags, exclusion of tags that were mapped to regions covered more than 10-times above average coverage and GC correction the average count of so called final tags decreased from 3 million and per sample to $2.160\pm0.065$ million and $2.099\pm0.221$ million of tags for IonTorrent PGM and MiSeq, respectively (for more details see *Minarik et al.*, *2015).*

**Example 2**

***Procedure of sample preparation for MiSeq (Illumina Inc., San Diego, CA, USA)) sequencer***

Complete method of obtaining the sequence data is described bellow as simple list of the main procedures since mostly the standard routine laboratory procedures were used. Also the protocols for specific steps are well known to the persons skilled in the art of molecular biology, prenatal testing and bioinformatics and such a person is aware of the possible modifications of the procedures. Many special protocols are also provided by the manufacturer of the kits or sequencing platform. More detailed description of laboratory procedures can be found in Minarik et al. 2015)

1. SAMPLE COLLECTION

The method was validated on samples from 12th week of pregnancy. 2 x 10mL blood sample was collected into K3-EDTA tubes, and processed in the next step within 48h.

2. PLASMA SEPARATION

Two stage plasma separation was carried out to allow optimal results of the test. Further details of the procedure can by found in Minarik et al. 2015.

3. DNA ISOLATION

Qiagen Blood Mini Kit was used. Plasma samples were either processed immediately or stored and processed within 1 month after plasma separation procedure with no observed effect on test results. Further details of the procedure can by found in Minarik et al. 2015.

4. LIBRARY PREPARATION

The procedure comprises following steps:

    a. MEASURING THE CONCENTRATION OF ISOLATED DNA
    b. END REPAIR
    c. SIZE SELECTION
    d. A-TAILING, ADAPTOR LIGATION
    e. PCR AMPLIFICATION
    f. LIBRARY QUANTITATION

5. SEQUENCING ANALYSIS

The method was validated with the following sequencing analysis settings:

Analysis type: FastQ only
Library type: Truseq LT
Paired end sequencing: Yes
Sequencing length: 2 x 100
Approximate sequencing run time: 24h

FastQ files are uploaded to the web service over Firefox web browser. Generated reports can later be downloaded using the same web service.

***Procedure with Ion Torrent platform***

In the processing of samples for Ion Torrent platform (Life Technologies Corp., San Francisco, CA, USA), steps 1 and 2 are necessarily identical, the other are similar or different, being specific this particular sequencing platform. Since the protocols are available from the producer or published elsewhere, the skilled person knows how to obtain sequence data using this platform.

**Example 3**

***Validation of the novel method with two sequencing platforms and comparison with other z-score methods***

Currently, there are several methods for trisomy detection based on z-score determination. We chose to compare the method according to the present invention, denoted MUL, with three most common methods already in use. We termed these methods based on the authors of articles describing them:

1. CHIU (method described in *Chiu et al.*, *2008*),
2. LAU (method described in *Lau et al.*, *2011*), and
3. SEH (method described in *Sehnert et al.*, *2011*)

The CHIU method, briefly summarized, is based on the fractional genomic representation (FGR) of the chromosome 21, which is a ratio $r_{21}$ of the number of tags mapped (after GC correction) to chromosome 21 to the total number of mapped tags (after GC correction and excluding tags mapped to sex chromosomes). The mean and standard deviation of $r_{21}$ of the control samples was used to normalize the $r_{21}$ value of a sample with unknown karyotype

$$z_{21} = \frac{r_{21} - E[r_{21}|\text{normal control}]}{\sqrt{var[r_{21}|\text{normal control}]}},$$

and if the normalized value $z_{21}$ is greater than 3, the sample is classified as trisomic (this is because *Chiu et al.*, *2008* assumed that the normalized values follow the standard normal distribution).

*Sehnert et al.*, *2011* and *Lau et al.*, *2012* proposed a different algorithm that calculates the ratio $q_k$ of the number of tags mapped to chromosome 21 to the number of tags mapped to some other chromosome (so called internal reference chromosome). While *Sehnert et al.*, *2011* chose chromosome 9 to be the optimal internal reference for the chromosome 21, *Lau et al., 2012* chose it to be chromosome 14. As was the case with CHIU method, the mean and standard deviation of $q_{21}$ of the control samples was used to normalize the $q_{21}$ value of a sample with unknown karyotype

$$\zeta_{21} = \frac{q_{21} - E[q_{21}|\text{normal control}]}{\sqrt{var[q_{21}|\text{normal control}]}},$$

and if the normalized value $\zeta_{21}$ is greater than 3, the sample is classified as trisomic (it was again assumed by *Sehnert et al.*, *2011* and *Lau et al., 2012* that the normalized values $\zeta$ follow the standard normal distribution).

For all z-score methods, including the MUL method according to the present invention (schematically depicted on Fig. 2), the input tag count data were same (see above THE METHOD, TRAINING SAMPLES, without optional size selection step). The training set consisted of 60 euploid samples same for all methods. Of the rest of the 71 test samples, there were 24 T21 cases and 47 euploid cases. Additionally, the internal reference bins IRB for our method MUL were found as described above (see THE METHOD, TRAINING SAMPLES). Tab. 1 displays z-scores of T21 samples from our T21 test set (the highest values are in bold). With the cut-off value 3 no false negatives were observed in any of the discussed methods.

Observed differences in z-scores between MiSeq and IonTorrent can be attributed to differences between biological sample processing, machine biases or other unknown factors.

Further, table 2 demonstrates results for 6 samples with trisomy of chromosome 18 and table 3 demonstrates results for 5 samples with trisomy of chromosome 13. Sequence data for T18 and T13 samples were obtained using MiSeq platform.

Furthermore, one sample with monosomy of chromosome 18 was analyzed. The results are demonstrated in the table 4. Z-score values are negative in the case of monosomy since in this case one copy of the chromosome 18 is missing. As a result, the amount of tags mapped the chromosome 18 is less than in normal samples with two copies of chromosome 18, what the value of z-score demonstrates.

Naturally, no far reaching conclusion cannot be deduced from one experiment, however, the example demonstrated that monosomy can be detected by our method at least as well as with prior art methods.

As it is demonstrated in the tables 1 to 4, z-score values of trisomic samples determined by the method of the present invention are significantly higher than values determined by any of the prior art method. For example, for T21 trisomic samples we obtained median difference of z-score values -1.895, -1.595, -2.485 when comparing CHIU, SEH and LAU, respectively, with our method with difference $p < 0.05$ for all prior art according to Dunn's multiple comparisons test), while values of normal (non-trisomic) samples are substantially identical (with respect to chromosome 21 we obtained median difference from our method -0.15, -0.05, -0.21 for CHIU, SEH and LAU, respectively), which means that the distance of z-scores of trisomic samples from the cut-off value is for the present invention increased when compared with the prior art methods while the distance of the euploid samples from this cut-off value is basically preserved.

**Table 1:** Trisomic T21 samples on MiSeq and IonTorrent. All samples were correctly classified by each method at the cut-off level 3. The highest value of z-score (marked in bold font) for each sample and machine were achieved by the MUL method according to the present invention. Each row corresponds with one biological sample

| MiSeq z-score | | | | IonTorrent z-score | | | |
|---|---|---|---|---|---|---|---|
| CHIU | LAU | SEH | MUL | CHIU | LAU | SEH | MUL |
| 31.13 | 28.6 | 28.64 | **37.94** | 27.25 | 27.3 | 27.12 | **33.79** |
| 19.01 | 17.8 | 17.15 | **22.43** | 18.26 | 17.47 | 16.35 | **22.39** |
| 17.77 | 16.15 | 16.12 | **20.09** | 15.51 | 14.8 | 14.35 | **18.43** |
| 14.99 | 14.14 | 13.57 | **18.34** | 13.28 | 13.27 | 12.31 | **15.05** |
| 14.21 | 13.13 | 12.51 | **16.48** | 12.95 | 13.03 | 11.6 | **15.98** |
| 12.45 | 12.14 | 11.58 | **15.28** | 13.08 | 12.34 | 12.59 | **16.07** |
| 11.88 | 10.8 | 10.85 | **14.79** | 12.64 | 12.85 | 12.25 | **15.36** |
| 12.01 | 10.78 | 9.77 | **14.64** | 9.72 | 9.47 | 9.29 | **11.69** |
| 10.43 | 9.36 | 9.43 | **12.79** | 11.56 | 12.17 | 10.52 | **13.67** |
| 10.56 | 9.55 | 9 | **10.68** | 8.57 | 7.77 | 8.44 | **10.91** |
| 9.87 | 9.05 | 8.47 | **11.91** | 6.87 | 6.77 | 6.07 | **8.6** |
| 9.44 | 9.55 | 9.11 | **10.77** | 10.49 | 10.68 | 9.07 | **12.71** |
| 9.43 | 8.75 | 8.8 | **11.13** | 10.56 | 11.1 | 9.88 | **12.36** |
| 8.87 | 8.25 | 8.58 | **10.84** | 8.14 | 7.59 | 7.68 | **9.47** |
| 8.4 | 7.71 | 7.95 | **9.77** | 5.45 | 6.03 | 5.34 | **7.28** |
| 8.45 | 7.88 | 7.87 | **10.63** | 8.23 | 7.94 | 7.65 | **9.98** |
| 8.8 | 8.73 | 7.55 | **9.42** | 6.94 | 7.17 | 7.59 | **8.5** |
| 8.13 | 8.08 | 7.07 | **9.95** | 7.71 | 6.73 | 7.92 | **9.99** |
| 8.41 | 7.66 | 7.43 | **9.78** | 6.88 | 7.03 | 5.59 | **8.61** |
| 8.15 | 8.29 | 7.76 | **9.71** | 7.21 | 6.36 | 6.75 | **9.56** |
| 7.28 | 7.15 | 6.31 | **8.75** | 5.76 | 5.58 | 5.53 | **7.12** |
| 7.02 | 6.31 | 6.91 | **8.28** | 5.91 | 6.35 | 5.94 | **6.78** |
| 7.15 | 6.25 | 6.13 | **8.38** | 5.96 | 5.23 | 5.58 | **6.48** |
| 5.02 | 5.48 | 4 | **6.32** | 6.12 | 5.93 | 5.58 | **8.15** |

**Table 2:** Z-score for T18 (MiSeq, 6 samples)

| CHIU | LAU | SEH | MUL |
|------|------|-------|-------|
| 17,9 | 15,5 | 17,65 | **22,2** |
| 13,36 | 10,54 | 11,82 | **13,8** |
| 9,82 | 8,53 | 9,79 | **16,54** |
| 9,42 | 8,57 | 9,29 | **10,78** |
| 5,73 | 4,75 | 5,39 | **6,63** |
| 7,23 | 6,35 | 8,78 | **9,83** |

**Table 3:** Z-score for T13 (MiSeq, 5 samples)

| CHIU | LAU | SEH | MUL |
|-------|-------|-------|-------|
| 22,59 | 21,88 | 26,46 | **30,73** |
| 16,91 | 16,8 | 19,76 | **25,47** |
| 22,98 | 22,16 | 26,56 | **31,76** |
| 21,41 | 20,4 | 24,65 | **24,86** |
| 3,92 | 4,75 | 4,79 | **6,44** |

**Table 4:** Z-score for monosomic chromosome 18 (MiSeq, 1 sample)

| CHIU | LAU | SEH | MUL |
|--------|--------|--------|--------|
| -17,34 | -12,67 | -18,67 | -16,14 |

**REFERENCES**

**Non-patent literature**

Bianchi, D.W., L.D. Platt, J.D. Goldberg, A.Z. Abuhamad, A.J. Sehnert, and R.P. Rava, Genome-wide fetal aneuploidy detection by maternal plasma DNA sequencing. Obstet Gynecol, 2012. 119(5): p. 890-901.

Chiu, R. W., K. A. Chan, Y. Gao, V. Y. Lau, W. Zheng, T. Y. Leung, C. H. Foo, B. Xie, N. B. Tsui, F. M. Lun, et al., Noninvasive prenatal diagnosis of fetal chromosomal aneuploidy by massively parallel genomic sequencing of dna in maternal plasma. Proceedings of the National Academy of Sciences, 105(51):20458-20463, 2008.

Chiu, R.W., R. Akolekar, Y.W. Zheng, T.Y. Leung, H. Sun, K.C. Chan, F.M. Lun, A.T. Go, E.T. Lau, W.W. To, W.C. Leung, R.Y. Tang, S.K. Au-Yeung, H. Lam, Y.Y. Kung, X. Zhang, J.M. van Vugt, R. Minekawa, M.H. Tang, J. Wang, C.B. Oudejans, T.K. Lau, K.H. Nicolaides, and Y.M. Lo, Non-invasive prenatal assessment of trisomy 21 by multiplexed maternal plasma DNA sequencing: large scale validity study. BMJ, 2011. 342: p. c7401.

Ehrich, M., C. Deciu, T. Zwiefelhofer, J.A. Tynan, L. Cagasan, R. Tim, V. Lu, R. McCullough, E. McCarthy, A.O. Nygren, J. Dean, L. Tang, D. Hutchison, T. Lu, H. Wang, V. Angkachatchai, P. Oeth, C.R. Cantor, A. Bombard, and D. van den Boom, Noninvasive detection of fetal trisomy 21 by sequencing of DNA in maternal blood: a study in a clinical setting. Am J Obstet Gynecol, 2011. 204(3): p. 205 e1-11.

Fan, H.C., Y.J. Blumenfeld, U. Chitkara, L. Hudgins, and S.R. Quake, Noninvasive diagnosis of fetal aneuploidy by shotgun sequencing DNA from maternal blood. Proc Natl Acad Sci USA, 2008. 105(42): p. 16266-71.

Fortin, F.-A., et al. "DEAP: Evolutionary algorithms made easy." The Journal of Machine Learning Research 13.1 (2012): 2171-2175.

Hudecova, I., D. Sahota, M. Heung, Y. Jin, W.S. Lee, T.Y. Leung, Y.M.D. Lo, and R.W. Chiu., Maternal plasma fetal dna fractions in pregnancies with low and high risks for fetal chromosomal aneuploidies. PloS one, 9(2):e88484, 2014.

Langmead, B., Salzberg, S., Fast gapped-read alignment with Bowtie 2. Nature Methods. 2012, 9:357-359

Lau, T. K., F. Chen, X. Pan, R.K. Pooh, F. Jiang, Y. Li, H. Jiang, X. Li, S. Chen, and X. Zhang. Noninvasive prenatal diagnosis of common fetal chromosomal aneuploidies by maternal plasma DNA sequencing. Journal of Maternal-Fetal and Neonatal Medicine, 25(8): 1370{ 1374, 2012.

Liao, C., A.H. Yin, C.F. Peng, F. Fu, J.X. Yang, R. Li, Y.Y. Chen, D.H. Luo, Y.L. Zhang, Y.M. Ou, J. Li, J. Wu, M.Q. Mai, R. Hou, F. Wu, H. Luo, D.Z. Li, H.L. Liu, X.Z. Zhang, and K. Zhang, Noninvasive prenatal diagnosis of common aneuploidies by semiconductor sequencing. Proc Natl Acad Sci USA, 2014. 111(20): p. 7415-20.

Lo, Y.M., N. Corbetta, P.F. Chamberlain, V. Rai, I.L. Sargent, C.W. Redman, and J.S. Wainscoat, Presence of fetal DNA in maternal plasma and serum. Lancet, 1997. 350(9076): p. 485-7.

Rava, R.P., A. Srinivasan, A.J. Sehnert, and D.W. Bianchi. Circulating fetal cell-free DNA fractions differ in autosomal aneuploidies and monosomy X. Clinical chemistry, 60(1):243-250, 2014.

Minarik, G., Repiska, G., Hyblova, M., Nagyova, E., Soltys, K., Budis, J. et al. Utilization of Benchtop Next Generation Sequencing Platforms Ion Torrent PGM and MiSeq in Noninvasive Prenatal Testing for Chromosome 21 Trisomy and Testing of Impact of In Silico and Physical Size Selection on Its Analytical Performance. PLoS One. 2015;10(12):e0144811. doi: 10.1371/journal.pone.0144811.

Sehnert, A.J., B. Rhees, D. Comstock, E. de Feo, G. Heilek, J. Burke, and R.P. Rava, Optimal detection of fetal chromosomal abnormalities by massively parallel DNA sequencing of cell-free fetal DNA from maternal blood. Clin Chem, 2011. 57(7): p. 1042-9.

Yu, S.C. et al., Size-based molecular diagnostics using plasma DNA for noninvasive prenatal testing. Proceedings of the National Academy of Sciences 111.23 (2014): 8583-8588.

**Patent documents**

EP 2183693

EP 2366031

US 8296076

WO 2011/051283

**Claims**

1. A method for determining fetal aneuploidy of chromosome of interest from a maternal blood sample comprising a mixture of cell-free DNA molecules of fetal and maternal origin, said method comprising:

   a) isolation of cell-free DNA from maternal blood;
   b) performing a random sequencing on at least a portion of a plurality of the cell-free DNA molecules contained in the blood, the portion comprising DNA corresponding to at least one chromosome of interest and a plurality of presumably euploid chromosomes, thereby obtaining plurality of sequence reads;
   c) mapping the sequence reads to the reference human genome, thereby obtaining plurality of mapped tags;
   d) splitting said reference human genome into 1Mbp subsequent and non-overlapping bins;
   e) determination of distribution of the tags to said bins and whole chromosomes according to their mapping location;

f) determining fractional bin value *FB* as a ratio of number of tags mapped to the bins for chromosome of interest in the sample to the number of tags mapped to internal reference bins IRB;

g) determining sample multinomial mean $\mu(n)$ and multinomial standard deviation $\sigma(n)$; wherein $\mu(n)$ and $\sigma(n)$ are calculated for a random variable defined as a ratio between number of tags mapped to the chromosome of interest in the sample and the sum of tags mapped to internal reference bins IRB, wherein the probability distribution of mapping of the tags to the chromosome of interest and to the internal reference bins IRB is multinomial, and the parameters of said multinomial distribution are determined from the training set of euploid blood samples;

h) determining z-score value as a difference between fractional bin value *FB* and multinomial mean $\mu(n)$ divided by multinomial standard deviation $\sigma(n)$; and

i) comparing z-score value to at least one cut-off value to determine whether an aneuploidy exists for the chromosome of interest;

wherein, as a prerequisite of the steps f)-i), a training set of euploid blood samples is subjected to essentially the same procedures consisting of steps a)-e) above and then the following steps are performed:

f') choosing internal reference autosomes IRA from candidate internal reference autosomes on the basis of the coefficient of variation CV thereof, while potentially aneuploid chromosomes are excluded;

g') choosing the internal reference bins IRB by an genetic algorithm from two candidate sets, the first candidate set being set of randomly selected bins, while bins corresponding to potentially aneuploid chromosomes are excluded, and the second candidate set being the set of bins corresponding to the best internal reference autosomes IRA determined in the previous step f'.

2. The method according to claim 1, where chromosome of interest is chromosome 8, 9, 13, 16, 18, 21, 22 and X.

3. The method according to claim 1 or 2, where aneuploidy is trisomy of chromosome 13, chromosome 18 or chromosome 21.

4. The method according to any one of claims 1-3, where training set of euploid blood samples comprises euploid male and euploid female samples.

5. The method according to any one of claims 1-4, further comprising size filtering step consisting in limitation of the size of reads acceptable for further processing.

6. The method according to any one of claims 1-5, where in choosing the internal reference autosomes IRA, for each potential internal reference combination the coefficient of variation CV is calculated, the candidate internal reference combinations of autosomes are ordered according to their CV value in increasing order and then internal reference combinations whose CV value is less than or equal to 1.1 multiple of the overall lowest CV value are chosen as IRA, wherein preferably the first hundred of these combinations are chosen as IRA for further processing.

7. The method according to any one of claims 1-6, where in choosing the internal reference bins IRB by a genetic algorithm the first candidate set is chosen from the candidate set on the basis of their *FB* values using Student range coefficient and at the same time on the basis of their CV values.

8. The method according to any one of claims 1-7 further comprising determination of fetal fraction.

9. The method according to claim 8 further comprising determination of the error of fetal fraction estimation.

10. The method according to any one of claims 1-9 further comprising determination of minimal tag count for reliable prediction of aneuploidy.

11. A computer program product comprising a computer readable medium comprising a plurality of instructions for controlling a computing system to perform all steps of the method according to any one of claims 1-10 following the step b) of performing a random sequencing.

12. A computer implemented method for determining fetal aneuploidy comprising all steps of the method according to any one of claims 1-10 following the step b) of performing a random sequencing.

**Patentansprüche**

1. Verfahren zur Bestimmung einer fetalen Aneuploidie eines Chromosoms von Interesse aus einer mütterlichen Blutprobe, umfassend eine Mischung von zellfreien DNA-Molekülen fetalen und mütterlichen Ursprungs, wobei das Verfahren umfasst:

   a) Isolierung von zellfreier DNA aus mütterlichem Blut;
   b) Durchführen einer zufälligen Sequenzierung an mindestens einem Teil einer Vielzahl der zellfreien DNA-Moleküle, die in dem Blut enthalten sind, wobei der Teil DNA umfasst, die mindestens einem Chromosom von Interesse und einer Mehrzahl von mutmaßlich euploiden Chromosomen entspricht, wodurch eine Vielzahl von Sequenz-Reads erhalten wird;
   c) Abbilden der Sequenz-Reads auf das menschliche Referenzgenom, wodurch eine Vielzahl von zugeordneten Markierungen erhalten wird;
   d) Aufteilen des menschlichen Referenzgenoms in 1 Mbp nachfolgende und nicht überlappende Abschnitte;
   e) Bestimmung einer Verteilung der Markierungen auf die Abschnitte und ganzen Chromosome entsprechend ihrer Zuordnungsstelle;
   f) Bestimmen eines anteiligen Abschnittwerts *FB* als ein Verhältnis von einer Anzahl von Markierungen, die den Abschnitten eines Chromosoms von Interesse in der Probe zugeordnet sind, zu der Anzahl von Markierungen, die internen Referenzabschnitten IRB zugeordnet sind;
   g) Bestimmen eines multinomialen Proben-mittelwerts $\mu(n)$ und einer multinomialen Standardabweichung $\sigma(n)$; wobei $\mu(n)$ und $\sigma(n)$ für eine Zufallsvariable berechnet werden, die definiert ist als ein Verhältnis zwischen einer Anzahl von Markierungen, die dem Chromosom von Interesse in der Probe zugeordnet sind und der Summe von Markierungen, die internen Referenzabschnitten IRB zugeordnet sind, wobei die Wahrscheinlichkeitsverteilung einer Zuordnung der Markierungen auf das Chromosom von Interesse und auf die internen Referenzabschnitte IRB multinomial ist, und die Parameter der multinomialen Verteilung aus dem Trainingssatz euploider Blutproben bestimmt werden;
   h) Bestimmen eines z-Score-Werts als eine Differenz zwischen einem anteiligen Abschnittwerts *FB* und einem multinomialen Mittelwert $\mu(n)$ dividiert durch eine multinomiale Standardabweichung $\sigma(n)$; und
   i) Vergleichen eines z-Score-Werts mit mindestens einem Grenzwert, um zu bestimmen, ob eine Aneuploidie für das Chromosom von Interesse existiert;

   wobei, als eine Voraussetzung für die Schritte f) - i), ein Trainingssatz euploider Blutproben im Wesentlichen den gleichen Prozeduren unterzogen wird, die aus den obigen Schritten a) - e) bestehen, und dann die folgenden Schritte durchgeführt werden:

   f') Auswahl interner Referenz-Autosomen IRA aus Kandidaten für interne Referenz-Autosomen auf der Basis von dessen Variationskoeffizienten *CV*, wogegen potentiell aneuploide Chromosomen ausgeschlossen werden;
   g') Auswählen der internen Referenzabschnitte IRB durch einen genetischen Algorithmus aus zwei Kandidatensätzen, wobei der erste Kandidatensatz ein Satz aus zufällig ausgewählten Abschnitten ist, wogegen Abschnitte, die potentiell aneuploiden Chromosomen entsprechen, ausgeschlossen werden, und der zweite Kandidatensatz der Satz von Abschnitten ist, der den besten internen Referenz-Autosomen IRA entspricht, die im vorherigen Schritt f' bestimmt wurden.

2. Verfahren nach Anspruch 1, wobei das Chromosom von Interesse Chromosom 8, 9, 13, 16, 18, 21, 22 und X ist.

3. Verfahren nach Anspruch 1 oder 2, wobei Aneuploidie eine Trisomie von Chromosom 13, Chromosom 18 oder Chromosom 21 ist.

4. Verfahren nach einem der Ansprüche 1 - 3, wobei der Trainingssatz euploider Blutproben euploide männliche und euploide weibliche Proben umfasst.

5. Verfahren nach einem der Ansprüche 1 - 4, ferner umfassend einen Größenfilterungsschritt, der darin besteht, die Größe von Reads zu begrenzen, die für die weitere Verarbeitung akzeptabel sind.

6. Verfahren nach einem der Ansprüche 1 - 5, wobei bei Auswahl der internen Referenz-Autosomen IRA für jede potentielle interne Referenzkombination der Variationskoeffizient *CV* berechnet wird, die Kandidaten für interne Referenzkombinationen von Autosomen gemäß ihres *CV-Werts* in aufsteigender Reihenfolge geordnet werden und dann interne Referenzkombinationen, deren CV-Wert kleiner oder gleich 1,1 mal dem insgesamt niedrigsten CV-Wert ist, als IRA gewählt werden, wobei vorzugsweise die ersten hundert dieser Kombinationen als IRA zur weiteren Verarbeitung gewählt werden.

7. Verfahren nach einem der Ansprüche 1 - 6, wobei bei der Auswahl der internen Referenzabschnitte IRB durch einen genetischen Algorithmus der erste Kandidatensatz aus dem Kandidatensatz auf der Grundlage ihrer *FB-Werte* unter Verwendung eines Student-Intervall-Koeffizienten und gleichzeitig auf Grundlage ihrer *CV-Werte* gewählt wird.

8. Verfahren nach einem der Ansprüche 1-7, ferner umfassend eine Bestimmung einer fetalen Fraktion.

9. Verfahren nach Anspruch 8, ferner umfassend eine Bestimmung des Fehlers einer fetalen Fraktionsschätzung.

10. Verfahren nach einem der Ansprüche 1 - 9, ferner umfassend eine Bestimmung einer minimalen Markierungsanzahl für eine zuverlässige Vorhersage einer Aneuploidie.

11. Computerprogrammprodukt, das ein computerlesbares Medium umfasst, das eine Vielzahl von Anweisungen zum Steuern / Regeln eines Computersystems umfasst, um alle Schritte des Verfahrens nach einem der Ansprüche 1 - 10 nach Schritt b), dem Durchführen einer zufälligen Sequenzierung, durchzuführen.

12. Computerimplementiertes Verfahren zum Bestimmen einer fetalen Aneuploidie, umfassend alle Schritte des Verfahrens nach einem der Ansprüche 1-10 nach Schritt b), dem Durchführen einer zufälligen Sequenzierung.

## Revendications

1. Procédé pour déterminer une aneuploïdie foetale d'un chromosome d'intérêt à partir d'un échantillon de sang maternel comprenant un mélange de molécules d'ADN acellulaire d'origine foetale et maternelle, ledit procédé comprenant :

   a) l'isolement d'ADN acellulaire à partir de sang maternel ;
   b) la réalisation d'un séquençage aléatoire sur au moins une partie d'une pluralité des molécules d'ADN acellulaire contenues dans le sang, la partie comprenant de l'ADN correspondant à au moins un chromosome d'intérêt et à une pluralité de chromosomes vraisemblablement euploïdes, en obtenant ainsi une pluralité de lectures de séquences ;
   c) la cartographie des lectures de séquences sur le génome humain de référence, en obtenant ainsi une pluralité de marqueurs cartographiés ;
   d) la division dudit génome humain de référence en bins successifs et non chevauchants de 1 Mpb ;
   e) la détermination de la distribution des marqueurs par rapport auxdits bins et aux chromosomes entiers en fonction de leur emplacement de cartographie ;
   f) la détermination de la valeur des bins fractionnels *FB* en tant que rapport du nombre de marqueurs cartographiés sur les bins pour le chromosome d'intérêt dans l'échantillon sur le nombre de marqueurs cartographiés sur des bins de référence internes IRB ;
   g) la détermination de la moyenne mutinomiale de l'échantillon $\mu(n)$ et de l'écart-type multinomial $\sigma(n)$ ; où $\mu(n)$ et $\sigma(n)$ sont calculés pour une variable aléatoire définie comme un rapport entre le nombre de marqueurs cartographiés sur le chromosome d'intérêt dans l'échantillon et la somme des marqueurs cartographiés sur les bins de référence internes IRB, où la distribution de probabilité de cartographie des marqueurs sur le chromosome d'intérêt et sur les bins de référence internes IRB est multinomiale, et les paramètres de ladite distribution multinomiale sont déterminés à partir de l'ensemble d'entraînement d'échantillons de sang euploïdes ;
   h) la détermination de la valeur du score z en tant que différence entre la valeur des bins fractionnels *FB* et la moyenne multinomiale $\mu(n)$ divisée par l'écart-type multinomial $\sigma(n)$ ; et
   i) la comparaison de la valeur du score z à au moins une valeur seuil afin de déterminer s'il existe une aneuploïdie pour le chromosome d'intérêt ;

   dans lequel, en tant que prérequis des étapes f)-i), un ensemble d'entraînement d'échantillons de sang euploïdes est soumis à essentiellement les mêmes procédures consistant en les étapes a)-e) ci-dessus puis les étapes suivantes sont réalisées :

   f') le choix d'autosomes de référence internes IRA à partir d'autosomes de référence internes candidats en se basant sur le coefficient de variation CV de ceux-ci, alors que les chromosomes potentiellement aneuploïdes sont exclus ;

g') le choix de bins de référence internes IRB par un algorithme génétique à partir de deux ensembles candidats, le premier ensemble candidat étant un ensemble de bins sélectionnés de manière aléatoire, alors que les bins correspondant à des chromosomes potentiellement aneuploïdes sont exclus, et le second ensemble candidat étant l'ensemble de bins correspondant aux meilleurs autosomes de référence internes IRA déterminés à l'étape f' précédente.

2. Procédé selon la revendication 1, dans lequel le chromosome d'intérêt est le chromosome 8, 9, 13, 16, 18, 21, 22 et X.

3. Procédé selon la revendication 1 ou 2, dans lequel l'aneuploïdie est une trisomie du chromosome 13, chromosome 18 ou chromosome 21.

4. Procédé selon l'une quelconque des revendications 1 à 3, dans lequel l'ensemble d'entraînement d'échantillons de sang euploïdes comprend des échantillons masculins euploïdes et féminins euploïdes.

5. Procédé selon l'une quelconque des revendications 1 à 4, comprenant en outre une étape de filtrage de taille consistant en la limitation de la taille des lectures acceptables pour un traitement supplémentaire.

6. Procédé selon l'une quelconque des revendications 1 à 5, dans lequel lors du choix des autosomes de référence internes IRA, pour chaque combinaison de référence interne potentielle, le coefficient de variation CV est calculé, les combinaisons de référence internes candidates des autosomes sont ordonnées en fonction de leur valeur de CV par ordre croissant puis les combinaisons de référence internes dont la valeur de CV est inférieure ou égale à 1,1 multiple de la valeur de CV la plus faible globale sont choisies en tant qu'IRA, où de préférence la première centaine de ces combinaisons sont choisies en tant qu'IRA pour un traitement supplémentaire.

7. Procédé selon l'une quelconque des revendications 1 à 6, dans lequel lors du choix des bins de référence internes IRB par un algorithme génétique, le premier ensemble candidat est choisi à partir de l'ensemble candidat en se basant sur leurs valeurs de *FB* en utilisant le coefficient de l'intervalle de Student et, en même temps, en se basant sur leurs valeurs de *CV*.

8. Procédé selon l'une quelconque des revendications 1 à 7, comprenant en outre la détermination de la fraction foetale.

9. Procédé selon la revendication 8, comprenant en outre la détermination de l'erreur de l'estimation de la fraction foetale.

10. Procédé selon l'une quelconque des revendications 1 à 9, comprenant en outre la détermination du nombre de marqueurs minimal pour la prédiction fiable d'une aneuploïdie.

11. Produit de programme informatique comprenant un support lisible par ordinateur comprenant une pluralité d'instructions pour contrôler un système informatique afin de réaliser toutes les étapes du procédé selon l'une quelconque des revendications 1 à 10 après l'étape b) de réalisation d'un séquençage aléatoire.

12. Procédé mis en oeuvre par ordinateur pour déterminer une aneuploïdie foetale comprenant toutes les étapes du procédé selon l'une quelconque des revendications 1 à 10 après l'étape b) de réalisation d'un séquençage aléatoire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2183693 A
- EP 2366031 A
- US 8296076 B
- WO 2011051283 A, Benz, M.

### Non-patent literature cited in the description

- BIANCHI, D.W. ; L.D. PLATT ; J.D. GOLDBERG ; A.Z. ABUHAMAD ; A.J. SEHNERT ; R.P. RAVA. Genome-wide fetal aneuploidy detection by maternal plasma DNA sequencing. *Obstet Gynecol,* 2012, vol. 119 (5), 890-901
- CHIU, R. W. ; K. A. CHAN ; Y. GAO ; V. Y. LAU ; W. ZHENG ; T. Y. LEUNG ; C. H. FOO ; B. XIE ; N. B. TSUI ; F. M. LUN et al. Noninvasive prenatal diagnosis of fetal chromosomal aneuploidy by massively parallel genomic sequencing of dna in maternal plasma. *Proceedings of the National Academy of Sciences,* 2008, vol. 105 (51), 20458-20463
- CHIU, R.W. ; R. AKOLEKAR ; Y.W. ZHENG ; T.Y. LEUNG ; H. SUN ; K.C. CHAN ; F.M. LUN ; A.T. GO ; E.T. LAU ; W.W. TO. Non-invasive prenatal assessment of trisomy 21 by multiplexed maternal plasma DNA sequencing: large scale validity study. *BMJ,* 2011, vol. 342, c7401
- EHRICH, M. ; C. DECIU ; T. ZWIEFELHOFER ; J.A. TYNAN ; L. CAGASAN ; R. TIM ; V. LU ; R. MCCULLOUGH ; E. MCCARTHY ; A.O. NYGREN. Noninvasive detection of fetal trisomy 21 by sequencing of DNA in maternal blood: a study in a clinical setting. *Am J Obstet Gynecol,* 2011, vol. 204 (3), 205 e1-11
- FAN, H.C. ; Y.J. BLUMENFELD ; U. CHITKARA ; L. HUDGINS ; S.R. QUAKE. Noninvasive diagnosis of fetal aneuploidy by shotgun sequencing DNA from maternal blood. *Proc Natl Acad Sci USA,* 2008, vol. 105 (42), 16266-71
- FORTIN, F.-A. et al. DEAP: Evolutionary algorithms made easy. *The Journal of Machine Learning Research,* 2012, vol. 13.1, 2171-2175
- HUDECOVA, I. ; D. SAHOTA ; M. HEUNG ; Y. JIN ; W.S. LEE ; T.Y. LEUNG ; Y.M.D. LO ; R.W. CHIU. Maternal plasma fetal dna fractions in pregnancies with low and high risks for fetal chromosomal aneuploidies. *PloS one,* 2014, vol. 9 (2), e88484
- LANGMEAD, B. ; SALZBERG, S. Fast gapped-read alignment with Bowtie 2. *Nature Methods,* 2012, vol. 9, 357-359
- LAU, T. K. ; F. CHEN ; X. PAN ; R.K. POOH ; F. JIANG ; Y. LI ; H. JIANG ; X. LI ; S. CHEN ; X. ZHANG. Noninvasive prenatal diagnosis of common fetal chromosomal aneuploidies by maternal plasma DNA sequencing. *Journal of Maternal-Fetal and Neonatal Medicine,* 2012, vol. 25 (8), 1370-1374
- LIAO, C. ; A.H. YIN ; C.F. PENG ; F. FU ; J.X. YANG ; R. LI ; Y.Y. CHEN ; D.H. LUO ; Y.L. ZHANG ; Y.M. OU. Noninvasive prenatal diagnosis of common aneuploidies by semiconductor sequencing. *Proc Natl Acad Sci USA,* 2014, vol. 111 (20), 7415-20
- LO, Y.M. ; N. CORBETTA ; P.F. CHAMBERLAIN ; V. RAI ; I.L. SARGENT ; C.W. REDMAN ; J.S. WAINSCOAT. Presence of fetal DNA in maternal plasma and serum. *Lancet,* 1997, vol. 350 (9076), 485-7
- RAVA, R.P. ; A. SRINIVASAN ; A.J. SEHNERT ; D.W. BIANCHI. Circulating fetal cell-free DNA fractions differ in autosomal aneuploidies and monosomy X. *Clinical chemistry,* 2014, vol. 60 (1), 243-250
- MINARIK, G. ; REPISKA, G. ; HYBLOVA, M. ; NAGYOVA, E. ; SOLTYS, K. ; BUDIS, J. et al. Utilization of Benchtop Next Generation Sequencing Platforms Ion Torrent PGM and MiSeq in Noninvasive Prenatal Testing for Chromosome 21 Trisomy and Testing of Impact of In Silico and Physical Size Selection on Its Analytical Performance. *PLoS One,* 2015, vol. 10 (12), e0144811
- SEHNERT, A.J. ; B. RHEES ; D. COMSTOCK ; E. DE FEO ; G. HEILEK ; J. BURKE ; R.P. RAVA. Optimal detection of fetal chromosomal abnormalities by massively parallel DNA sequencing of cell-free fetal DNA from maternal blood. *Clin Chem,* 2011, vol. 57 (7), 1042-9
- YU, S.C. et al. Size-based molecular diagnostics using plasma DNA for noninvasive prenatal testing. *Proceedings of the National Academy of Sciences,* 2014, vol. 111.23, 8583-8588